(19) Europäisches Patentamt
European Patent Office
Office européen des brevets

(11) **EP 4 190 378 A1**

(12) **EUROPEAN PATENT APPLICATION**

(43) Date of publication:
**07.06.2023 Bulletin 2023/23**

(21) Application number: **21212446.5**

(22) Date of filing: **06.12.2021**

(51) International Patent Classification (IPC):
**A61M 5/315** (2006.01)  **A61J 1/06** (2006.01)

(52) Cooperative Patent Classification (CPC):
**A61M 5/315**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Becton, Dickinson and Company**
**Franklin Lakes, NJ 07417-1880 (US)**

(72) Inventors:
• **LEHEE, Guillaume**
**38340 Voreppe (FR)**
• **FREMON, Benoit**
**38410 Saint Martin d'Uriage (FR)**

(74) Representative: **Germain Maureau**
**12, rue Boileau**
**69006 Lyon (FR)**

(54) **STOPPER FOR A SYRINGE HAVING ANTI-STICKING FEATURES**

(57) A stopper for a syringe including a body having a tail adapted for attachment to a plunger of the syringe, a head, and, optionally, a plurality of annular ribs and grooves around the circumference of the outer sidewall of the body, where the ribs are separated by the grooves. The stopper includes a plurality of protrusions extending from an outer surface of the tail and/or the head and/or a plurality of protrusions extending outwardly from the outer sidewall from within at least one groove. The protrusions extending from an outer surface of the tail and/or the head are arranged in at least one circle, and an angular distance between two adjacent protrusions in the circle is less than the angular distance occupied by one of the protrusions. Also, a syringe including such a stopper.

FIG.5

**Description**

BACKGROUND OF THE INVENTION

Field of the Invention

The present invention is directed to a stopper for a syringe having anti-sticking features extending from the outer surface of the stopper body and a syringe including such a stopper.

Description of Related Art

With prior art stoppers for syringes, especially for stoppers made from rubber or rubber-like materials, during storage or assembly, a surface of a first stopper may stick with a surface of a second stopper. For instance, the outer surface of the tail and/or the head of one stopper can become stuck to the outer surfaces of the tail, the head, or the sidewall of another stopper or the outer sidewalls of two stoppers can stick together. Sticking can occur when the stoppers are packaged in plastic bags that may be evacuated of air and stacked on a pallet during storage or shipping. Sticking can also happen during the production of syringes, when the stoppers are conveyed in a vibrating bowl prior to being assembled into the syringe. Thus, there is a need for stoppers that decrease both the occurrence of sticking in these situations and the bonding strength of any sticking that does occur.

For non-coated stoppers, sticking of the stoppers to one another is caused by intramolecular forces, such as covalent bonding, which can be promoted by gamma sterilization, and/or intermolecular forces such Van der Waals forces. Covalent bonding requires proximity on the angstrom scale ($10^{-10}$ m) between two surfaces of two different stoppers to allow formation of the covalent bonds, for example, carbon sulfur bonds (Penocchio et al., Structural Features of the Carbon- Sulfur Chemical Bond: A Semi-Experimental Perspective, Can. J. Chem., 2016, Vol. 94 No. 12, pp. 1065-1076). Van der Waals forces vary with the inverse of the power of six of the distance between the two surfaces where the force is exerted (Israelachvili, Intermolecular and Surfaces Forces, 2003), thus requiring very close contact at the interface to effectively obtain an interaction between the two surfaces.

Thus, intimate contact between the surfaces of the stoppers is needed for the stoppers to macroscopically stick to one another.

Close contact, which will be referred to herein as the apparent contact area, between two surfaces of the two different stoppers can occur when an external force is applied to the surfaces of the stoppers that are in contact with one another. This force can be applied to packaged stoppers, when air is evacuated from the bag containing the stoppers to increase packaging density, and when bags of stoppers are packaged in cardboard boxes that are stacked together on a pallet for shipping, the external force can be magnified.

In the case of elastic materials such as the materials used for stoppers, Hertz theory (Popov, Rigorous Treatment of Contact Problems - Hertzian Contact, Contact Mechanics and Friction, 2010; pp. 55-70) can be used to describe the increase of apparent contact area. While applicable on idealized surfaces (half plan/sphere, cylinder/cylinder, etc., with known parameters such as external constraint, Young modulus, Poisson ratio, etc.), numerical solving of the Hertz equation on more complicated geometries such as stoppers with anti-sticking features is not trivial. When adhesion (intramolecular forces) is taken into consideration (Johnson Kendall Roberts theory, Surface Energy and the Contact of Elastic Solids, Proc. R. Soc. London Math. Phys. Sci., 1971, vol. 324, no. 1558, pp. 301- 313), resolution on complex geometries is even more difficult.

Furthermore, the latest developments in contact mechanics (Multi Contact Mechanics, Persson, Theory of Rubber Friction and Contact Mechanics, J. Chem. Phys., 2001, vol. 115, no. 8, pp. 3840-3861) replaces the notion of apparent contact area by the effective contact area, which depends on the rugosity and chemical state at different lengths ranging from nanometer to millimeter scale. This makes approximation of the effective contact area with this approach only feasible by numerical simulation coupled with well characterized surfaces.

With these factors in mind, inventive stoppers having anti-sticking features were developed in order to reduce the sticking of stoppers to one another during assembly and storage.

SUMMARY OF THE INVENTION

The present invention is directed to a stopper for a syringe comprising a body having a proximal tail adapted for attachment to a plunger rod of the syringe and a distal head and a plurality of protrusions extending from an outer surface of the tail and/or the head. The protrusions are arranged in at least one circle on the outer surface of the tail and/or the head of the stopper and an angular distance between two adjacent protrusions in the circle is less than the angular distance occupied by one of the protrusions. The outer surfaces of the protrusions arranged in at least one circle define a polygonal structure. The protrusions may have any shape including, but not limited to protrusions having a cross-section that is circular, oval, square, polygonal, or in the shape of a letter or logo.

The body of the stopper may further include a cavity adapted to receive and engage at least a portion of the plunger rod of a syringe with an opening to the cavity provided in the tail of the stopper. The protrusions may be provided on the tail of the stopper and arranged in at least one circle on the outer surface of the tail of the stopper extending from an annular ring on the outer surface of the tail of the stopper defined between an outer perimeter of the outer surface and an outer perimeter of the cavity. When the protrusions are cylindrical and a single circle of protrusions is provided, a minimum number of protrusions provided in the circle is defined by:

$$n_{min} = int\left(\frac{\pi}{\theta_P}\right) + 1$$

where

$$\theta_P = 2tan^{-1}\frac{D_P}{\frac{1}{2}(D_O + D_C)}$$

where Dp is a diameter of each protrusion, $D_O$ is an outer diameter of the outer surface of the tail of the stopper and $D_C$ is a diameter of the outer perimeter of the cavity, and wherein the angular distance between protrusions is defined by:

$$\theta_S = (2\pi - (\theta_P \times n_{min}))/n_{min}$$

The protrusions may be arranged in an outer circle around an outer perimeter of the outer surface of the head of the stopper and with a protrusion within the outer circle positioned in a center of the outer surface of the head of the stopper.

The protrusions may be arranged in an outer circle around an outer perimeter of the outer surface of the tail and/or the head of the stopper and further in at least one additional concentric circle within the outer circle, and an angular distance between two adjacent protrusions in each circle is less than the angular distance occupied by one of the protrusions. The first outer circle of substantially cylindrical protrusions is provided on the tail and/or the head of the stopper adjacent an outer perimeter of the outer surface of the tail and/or the head of the stopper and a number n1 of protrusions provided in the first circle may be defined by:

$$n_1 > int\left(\frac{\pi}{tan^{-1}\frac{2\beta R}{R - \gamma R - \beta R}}\right) + 1$$

where R is an outer diameter of the outer surface of the tail and/or the head of the stopper, $\beta$ is a ratio of a radius r of each protrusion and the radius R (r/R), and $\gamma$ is a safety factor for a distance between the circle of protrusions and an outer perimeter of the end surface of the head of the stopper. Additional concentric circles of protrusions may be provided within the first circle of protrusions and a number $n_i$ of protrusions provided in the $i^{th}$ circle is defined by:

$$n_i > int\left(\frac{\pi}{tan^{-1}\frac{2\beta R}{R_i - \gamma R - \beta R}}\right) + 1$$

where $R_i$ is the inner diameter of the i-1 circle of protrusions.

The protrusions may be equally spaced around the at least one circle, the protrusions may have any shape including, but not limited to protrusions having a cross-section that is circular, oval, square, polygonal, or in the shape of a letter or logo, and/or the outer surface of the head of the stopper may be flat, convex, or conical. A convex outer surface of the head of the stopper is one in which the outer surface of the head of the stopper is curved, such that the apex of the curvature extends away from a plane which is orthogonal to the longitudinal axis of the stopper and comprising the outer perimeter of the outer surface, i.e., a distance between the apex of the outer surface of the head and the plane is greater than the distance between the portion of the outer surface of the head adjacent the outer perimeter of the outer surface of the head and the plane when the stopper is viewed from the side in a direction perpendicular to the longitudinal axis of the stopper. When the head of the stopper is convex, protrusions in a circle adjacent an outer perimeter of the outer surface of the head of the stopper may have a height in a direction extending outwardly from the outer surface of the head of the stopper that is greater than a height in a direction extending outwardly from the outer surface of the head of the stopper of protrusions located inside the circle adjacent an outer perimeter of the outer surface of the head of the stopper.

When the head of the stopper is conical, a circle of protrusions having a substantially triangular shape, preferably a shape that is substantially an isosceles triangle, with a vertex corresponding to the vertex angle of the triangular shape of the protrusion, a base corresponding to the base of the triangular shape of the protrusion, and two sides extending between the vertex and the base may extend from an outer surface of the head of the stopper.

The present invention is further directed to a stopper for a syringe comprising a body having a distal tail adapted for attachment to a plunger rod of the syringe, a distal head, a plurality of annular ribs around the circumference of an outer sidewall of the body, and a plurality of grooves around the circumference of an outer sidewall of the body, wherein the ribs are spaced from one another by the grooves and a plurality of protrusions extend outwardly from the outer sidewall of the body in a radial direction from within at least one of the grooves. An angular spacing of the protrusions within the at least one groove may be 30° or more, a median angular spacing of the protrusions within the at least one groove may be 40°-60°, and/or a maximum angular spacing of the protrusions within the at least one groove may be 60°-80°. A height of the protrusions in the radial direction may be 50% to less than 100% of the difference between a radius of the rib and a radius of the groove. Protrusions may be provided in more than one groove with an offset spacing from groove to groove corresponding to half the angular distance between the protrusions in each groove.

The present invention is additionally directed to a syringe comprising a syringe body defining a chamber, a plunger rod at least partially received within the chamber, and a stopper as discussed above attached to an end of the plunger received within chamber.

## BRIEF DESCRIPTION OF THE DRAWINGS

FIG. 1 is cross-sectional view of the inventive stopper within a syringe;

FIG. 2 is a cross-sectional view of an inventive stopper having anti-sticking protrusions extending from the outer surface of the tail;

FIG. 3 is a bottom view of the inventive stopper of FIG. 2;

FIG. 4 shows the contact between the tails of two of the inventive stoppers of FIG. 2 with the bottom of one of the inventive stoppers shown with solid lines and the bottom of the other inventive stopper shown with dashed lines;

FIG. 5 is a cross-sectional view of an inventive stopper having anti-sticking protrusions extending from the outer surface of the head;

FIG. 6 is a top view of the inventive stopper of FIG. 5;

FIG. 7 shows the contact between the heads of two of the inventive stoppers of FIG. 5 with the top of one of the inventive stoppers shown with solid lines and the top of the other inventive stopper shown with dashed lines;

FIG 8. shows the contact between the heads of two inventive stoppers that do not have an anti-sticking protrusion in the center of the outer surface of the head with the top of one of the inventive stoppers shown with solid lines and the top of the other inventive stopper shown with dashed lines;

FIG 9. shows the contact between the heads of two inventive stoppers that have an anti-sticking protrusion in the center of the outer surface of the head with the top of one of the inventive stoppers shown with solid lines and the top of the other inventive stopper shown with dashed lines;

FIG. 10 is a top view of an inventive stopper having concentric circles of anti-sticking protrusions extending from the outer surface of the head;

FIG. 11 is a graph showing the relationship between the maximum number $n_{max}$ of protrusions extending from the outer surface of the head of the inventive stopper and the ratio $\beta$ of the radius r of the protrusions to the radius R of the outer perimeter of the head of the stopper (r/R);

FIG. 12 is a graph showing the relationship between the maximum number of protrusions in each of the concentric circles of protrusions extending from the head of the inventive stopper and the ratio $\beta$ of the radius r of the protrusions to the radius R of the outer perimeter of the head of the stopper (r/R);

FIG. 13 is a graph showing the relationship between effective total contact area and the ratio β of the radius r of the protrusions to the radius R of the outer perimeter of the head of the stopper (r/R);

FIG. 14 is a bottom view of an inventive stopper having concentric circles of anti-sticking protrusions extending from the outer surface of the tail;

FIG. 15 is a cross-sectional view of an inventive stopper having anti-sticking protrusions extending from the outer surface of a convex head;

FIG. 16 is a cross-sectional view of an inventive stopper having anti-sticking protrusions extending from the outer surface of a conical head;

FIG. 17 is a top view of the inventive stopper of FIG. 16;

FIG. 18 is a cross-sectional view of an inventive stopper having anti-sticking protrusions extending from the outer sidewall of the stopper body;

FIG. 19 is a partial cross-sectional view of two prior art stoppers showing the contact between the outer sidewalls of the two stoppers with one of the stoppers shown with cross-hatching and one of the stoppers shown without cross-hatching;

FIG. 20 is a partial cross-sectional view of two of the inventive stoppers of FIG. 19 showing the contact between the outer sidewalls of the two inventive stoppers with one of the inventive stoppers shown with cross-hatching and one of the inventive stoppers shown without cross-hatching; and

FIG. 21 is a graph showing the angular spacing of the anti-sticking protrusions extending from the outer sidewall of the stopper body for a variety of stopper configurations.

DESCRIPTION OF THE INVENTION

As used herein, unless otherwise expressly specified, all numbers such as those expressing values, ranges, amounts or percentages may be read as if prefaced by the word "about", even if the term does not expressly appear. Any numerical range recited herein is intended to include all sub-ranges subsumed therein. For example, a range of "1 to 10" is intended to include any and all sub-ranges between and including the recited minimum value of 1 and the recited maximum value of 10, that is, all subranges beginning with a minimum value equal to or greater than 1 and ending with a maximum value equal to or less than 10, and all subranges in between, e.g., 1 to 6.3, or 5.5 to 10, or 2.7 to 6.1. Plural encompasses singular and vice versa. When ranges are given, any endpoints of those ranges and/or numbers within those ranges can be combined with the scope of the present invention. "Including", "such as", "for example" and like terms means "including/such as/for example but not limited to".

For purposes of the description hereinafter, spatial orientation terms, as used, shall relate to the referenced embodiment as it is oriented in the accompanying drawings, figures, or otherwise described in the following detailed description. However, it is to be understood that the embodiments described hereinafter may assume many alternative variations and configurations. It is also to be understood that the specific components, devices, features, and operational sequences illustrated in the accompanying drawings, figures, or otherwise described herein are simply exemplary and should not be considered as limiting. As used herein, "distal" refers the end of the stopper comprising the head or an injection end of the syringe. In the cross-sectional drawings of the stopper, "distal" refers to the top end of the stopper. Similarly, a "distal direction" is a direction toward the head of the stopper or toward the injection end of the syringe. As used herein, "proximal" refers the end of the stopper comprising the tail or a plunger end of the syringe. In the cross-sectional drawings, "proximal" refers to the bottom end of the stopper. Similarly, a "proximal direction" is a direction toward the tail of the stopper or toward the plunger end of the syringe.

The present invention is directed to a stopper 10 to be used in a syringe 12. The syringe comprises a syringe body 14 and a plunger 16 to which the stopper 10 is connected.

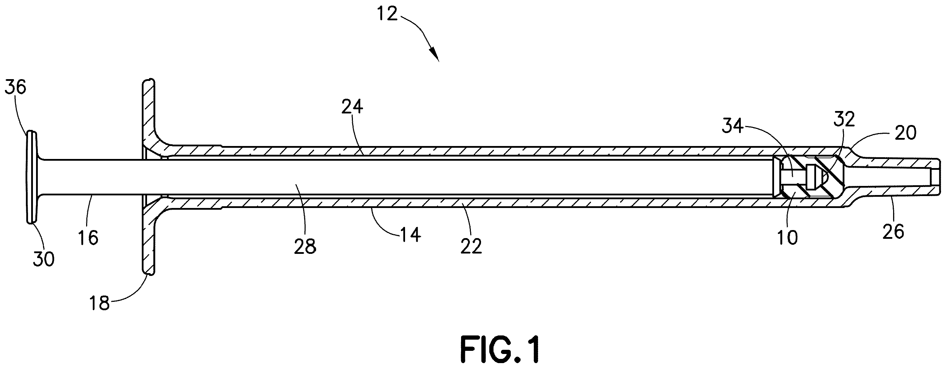

As shown in FIG. 1, the syringe body 14 comprises a proximal end 18, a distal end 20, and a sidewall 22 extending between the proximal end 18 and distal end 20. The sidewall 22 defines a chamber 24 adapted to receive a pharmaceutical composition. The chamber 24 is substantially cylindrical. A fitting 26 for attaching a cannula assembly to the syringe 12 extends in the longitudinal direction from the distal end 20 of the syringe body 14. The fitting 26 may be a luer lock or a luer slip. The cannula assembly may be permanently attached to the syringe 12 or, alternatively, cannula assembly may be assembled to the syringe 12 just before usage. The syringe body 14 may be made from plastic or glass.

The plunger 16 comprises a plunger rod 28 having a proximal end 30 and a distal end 32, an engagement portion 34 extending from the distal end 32 of the plunger rod 28, and may include a thumb pad 36 extending radially outward from the proximal end 30 of the plunger rod 28 in a direction substantially perpendicular to the longitudinal axis of the plunger rod 28. At least a portion of the plunger rod 28 and the engagement portion 34 of the plunger rod 28 are contained within the chamber 24 of the syringe body 14. The engagement portion 34 of the plunger rod 28 is adapted for connection to the stopper 10. The plunger 16 is movable with respect to the syringe body 14 such that a distal force applied to the plunger 16 while holding the syringe body 14 stationary causes the engagement portion 34 of the plunger rod 28 and the stopper 10 attached thereto to be displaced in a distal direction within the chamber 24 of the syringe body 14, and a proximal force applied to the plunger 16 while holding the syringe body 14 stationary causes the engagement portion 34 of the plunger rod 28 and the stopper 10 attached thereto to be displaced in a proximal direction within the chamber 24 of the syringe body.

At least a portion of the stopper 10 has a diameter that is equal to or larger than an inner diameter of the chamber 24 of the syringe body 14 thereby creating a sealing engagement between the portion of the stopper 10 and the inner surface of the sidewall 22 of the syringe body 14.

The inventive stopper 10 may be sized for used with any kind of syringes, such as standard 0.5 ml - 20 ml syringes.

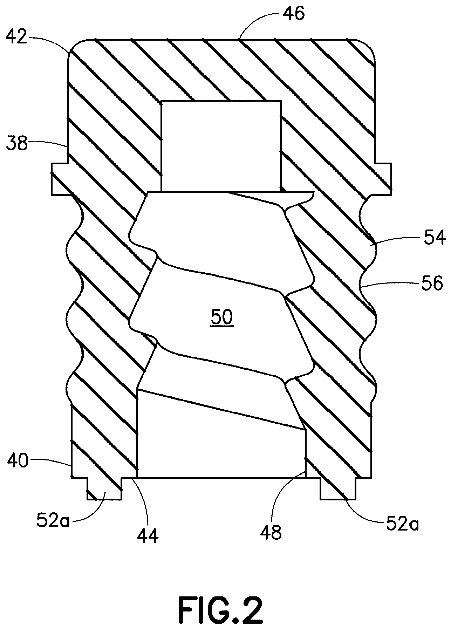

As shown in FIGS. 2, 3, 5, 6, and 9-17, the stopper 10 comprises a body 38 having a proximal tail 40 adapted for connection to the plunger rod 28 and a distal head 42 and anti-sticking features on at least one of the outer surface 44 of the tail 40 and the outer surface 46 of the head 42. The tail 40 includes an opening 48 to a cavity 50 defined within the body 38. The opening 48 and the cavity 50 are adapted to receive and engage the engagement portion 34 of the plunger rod 28 thereby attaching the stopper 10 to the plunger 16. The opening 48 of the cavity 50 may be a circular opening and have a diameter $D_C$ that is smaller than the outer diameter $D_O$ of the tail 40 of the stopper 10 such that the outer surface 44 of the tail 40 of the stopper 10 is an annular ring. While the stoppers 10 shown in the figures and discussed below include the cavity 50, it is to be understood that the cavity 50 is optional and the plunger 16 may be attached to the stopper 10 via other means, such that no cavity is required.

With prior art stoppers, during storage or assembly, the respective outer surfaces 44, 46 of the tail 40 and/or the head 42 of one stopper can become stuck to the outer surfaces 44, 46 of the tail 40 and/or the head 42 of another stopper, or to the sidewall of another stopper. The anti-sticking features of the inventive stopper 10 are provided to reduce the tendency of the faces 44, 46 of the tail 40 and/or the head 42 of one stopper 10 to stick to the faces 44, 46 of the tail 40 and/or the head 42 of another stopper 10.

The anti-sticking features may be protrusions 52 extending outwardly from the tail 40 and/or the head 42 of the stopper 10. While the protrusions 52 as shown in the figures and discussed below are substantially cylindrical having a substantially circular cross-section, the protrusions 52 may have any shape as long as the shape and dimensions of the protrusions 52 provides for anti-sticking of the tail 40 and/or head 42 of one stopper 10 to the tail 40 and/or head 42 of another stopper in a manner which will be described below. For example, the protrusions may have a cross-section that is circular, oval, square, polygonal, or in the shape of a letter or logo.

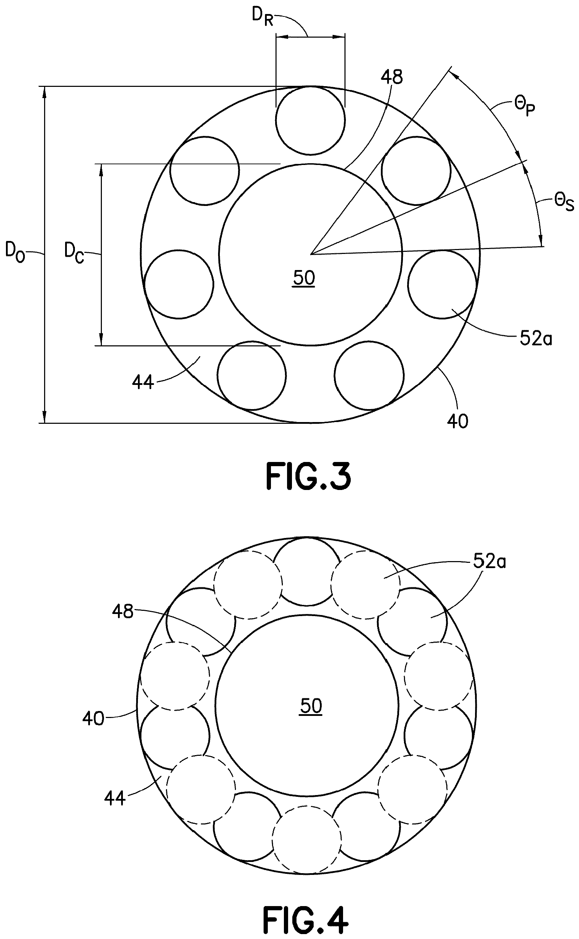

As shown in FIGS. 3, 6, 10, and 14 the protrusions 52 are arranged in at least one circle on the outer surface 44, 46 of the tail 40 and/or the head 42 of the stopper 10. The arrangement of the protrusions 52 on the circle defines a polygon. For example, three protrusions 52 may be arranged on the circle to define a triangle, four protrusions 52 may be arranged on the circle to define a square or rectangle, five protrusions 52 may be arranged on the square to define a pentagon, etc.

The angular distance $\theta_S$ between two adjacent protrusions 52 is less than the angular distance $\theta_P$ occupied by one of the protrusions 52, i.e., $\theta_S < \theta_P$. The angular distance $\theta_S$ between two adjacent protrusions 52 as used herein is the minimal angular distance between the outer surface of a protrusion 52 and the outer surface of the most adjacent protrusion 52. By dimensioning and positioning the protrusions 52 in this manner, the protrusions 52 on one stopper 10 cannot fit between the protrusions 52 of another stopper 10 and the maximum contact area between the outer surfaces 44, 46 of the tails 40 and/or the heads 42 of two stoppers 10 is limited to a maximum of the total surface area of the protrusions 52 of one of the stoppers 10.

With respect to the tail 40 of the stopper 10 (FIGS. 2 and 3), a plurality of protrusions 52a may extend outwardly in a proximal direction around the perimeter of the outer surface 44 of the tail 40 of the stopper 10. The protrusions 52a are equally spaced around the annular ring of the outer surface 44 of the tail 40.

The dimension of the protrusions 52a in the radial direction $D_R$ is less than or equal to the radial width of the annular ring as determined by the difference between the outer diameter Do of the outer surface 44 of the tail 40 of the stopper 10 and the diameter Dc of the outer perimeter of the opening 48 of the cavity 50. In the case of cylindrical protrusions 52a, the diameter of the protrusion 52a is less than or equal to the radial width of the annular ring as determined by the difference between the outer diameter $D_O$ of the tail 40 of the stopper 10 and the diameter $D_C$ of the outer perimeter of the opening 48 of the cavity 50.

The angular distance $\theta_S$ between two adjacent protrusions 52a is less than the angular distance $\theta_P$ occupied by one of the protrusions 52a, i.e., $\theta_S < \theta_P$. By dimensioning and positioning the protrusions 52a in this manner, the protrusions 52a on the outer surface 44 of the tail 40 of one stopper 10 cannot fit between the protrusions 52a on the outer surface 44 of the tail 40 of another stopper 10 (FIG. 4), and the maximum contact area between the outer surface 44 of the tails 40 of two stoppers 10 is limited to a maximum of the total surface area of the protrusions 52a of one of the stoppers 10.

The number of protrusions 52a can be calculated given the diameter Dp of the protrusions 52a extending around the circumference of the annular ring of the outer surface 44 of the tail 40 of the stopper 10, the outer diameter Do of the tail of the stopper, and the diameter $D_C$ of the opening 48 of the cavity 50.

When the protrusions 52a are radially centered within the annular ring of the outer surface 44 of the tail 40 of the stopper 10, i.e., the protrusions 52a are at a distance from the common origin of the outer perimeters of the tail 40 of the stopper 10 and the opening 48 of the cavity 50 that is the sum of the diameter $D_O$ of the tail 40 of the stopper 10 and the diameter $D_C$ of the outer perimeter of the opening 48 of the cavity 50 divided by 4 (($D_O$ + $D_C$)/4), the angular distance $\theta_P$ occupied by one protrusion 52a having a dimension (diameter) Dp extending around the circumference of the annular ring of the outer surface 44 of the tail 40 of the stopper 10, is:

$$\theta_P = 2tan^{-1} \frac{D_P}{\frac{1}{2}(D_O + D_C)}$$

Radially, the total angular distance occupied by all of the protrusions 52a is less than $\pi$. Thus, the minimum number $n_{min}$ of protrusions is:

$$n_{min} = int\left(\frac{\pi}{\theta_P}\right) + 1$$

and the angular distance $\theta_S$ between protrusions is:

$$\theta_S = (2\pi - (\theta_P \times n_{min})/n_{min}$$

As an example, when the maximum dimension (diameter) Dp extending around the circumference of the annular ring of the outer surface 44 of the tail 40 of the stopper 10 is 1 mm, the outer diameter Do of the tail of the stopper is 5mm, and the diameter Dc of the opening of the cavity is 3 mm.

$$\theta_P = 2tan^{-1} \frac{1}{\frac{1}{2}(5 + 3)} = 0.49\,rad$$

$$n_{min} = int\left(\frac{\pi}{0.49}\right) + 1 = 7$$

$$\theta_S = \frac{2\pi - 0.49 * 7}{7} = 0.4\,rad$$

In this configuration, the optimal number of protrusions is 7.

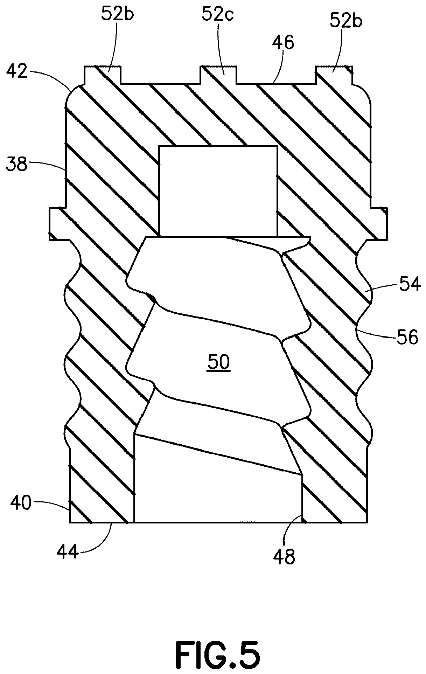

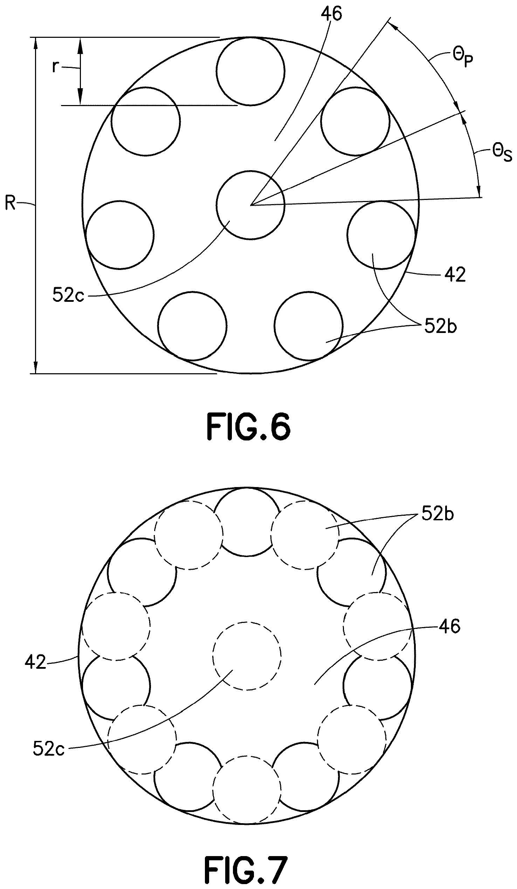

With respect to the head 42 of the stopper 10, as shown in FIGS. 5 and 6, a plurality of protrusions 52b extend outwardly in a distal direction from the outer surface 46 of the head 42. The protrusions 52b are provided in an area corresponding to the annular ring of the tail 40 and in a circle and equally spaced around the outer perimeter of the outer surface 46 of the head 42 of the stopper 10, such that the protrusions define a polygon, specifically in FIGS. 5 and 6, a heptagon. To minimize tail 40 to head 42 contact between two stoppers 10, the protrusions 52b on the outer surface 46 of the head 42 may be positioned in a corresponding manner to any protrusions 52a provided on the outer surface 44 of the tail 40 of the stopper with respect to the outer perimeter of the stopper 10 and the spacing around the outer perimeter of the outer surface 46 of the head 42 of the stopper 10.

The angular distance $\theta_S$ between two adjacent protrusions 52b is less than the angular distance $\theta_P$ occupied by one of the protrusions 52b, i.e., $\theta_S < \theta_P$. By dimensioning and positioning the protrusions 52b in this manner, the protrusions 52b on the outer surface 46 of the head 42 of one stopper 10 cannot fit between the protrusions 52b on the outer surface 46 of the head 42 of another stopper 10 (FIG. 7), and the maximum contact area between the outer surface 44 of the heads 42 of two stoppers 10 is limited to a maximum of the total surface area of the protrusions 52b of one of the stoppers 10.

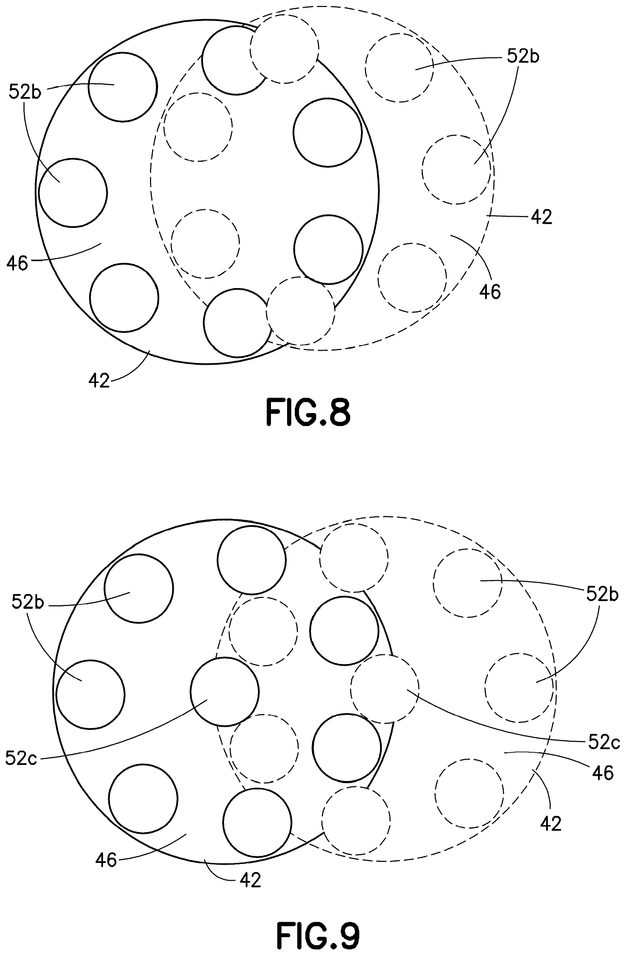

While such an arrangement of protrusions 52b on the outer surface 46 of the head 42 of the stopper 10 will reduce the contact area between the head 42 of one stopper 10 and the tail 40 of another stopper 10 and reduce the tendency of the stoppers 10 to stick to one another in the same manner that the protrusions 52a on the outer surface 44 of the tail 40 of the stopper 10 reduce the contact area between the tail 40 of one stopper 10 and the tail 40 of another stopper 10 and reduce the tendency of the tails 40 of the stoppers 10 to stick to one another, the contact area of the head 42 may not be sufficiently reduced and the potential for sticking may not be sufficiently reduced when head 42 to head 42 contact occurs between two stoppers 10 as shown in FIG. 8. In the configuration shown in FIG. 8, the two protrusions located at the intersection of the two stoppers 10 are in direct contact. This situation can be avoided by providing an additional protrusion 52c at the center of the outer surface 46 of the head 42 of the stopper 10, i.e., at the center of origin of the outer perimeter of the head 42 of the stopper 10, as shown in FIG. 9.

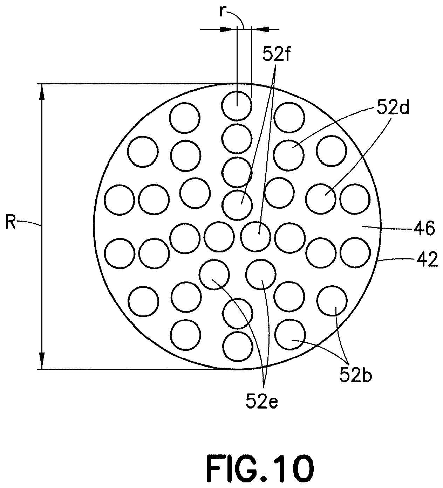

Alternatively or in addition, one or more further concentric circles of protrusions 52d, 52e having increasingly smaller diameters than the diameter of the circle of protrusions 52b provided around the outer perimeter of the outer surface 46 of the head 42 of the stopper 10 may be provided (FIG. 10). The number of these protrusions 52d, 52e and the placement of these protrusions 52d, 52e can be determined based on:

- the outer diameter R of the head 42 of the stopper 10,
- the maximum allowed direct rigid coverage $\alpha$,
- the radius r (half dimension) of the protrusions 52d, 52e,
- the ratio $\beta$ of the radius r of the protrusions 52d, 52e and the radius R of the outer diameter of the outer surface 46 of the head 42 of the stopper 10 (r/R),
- the number n of the protrusions 52b, 52d, 52e, 52f on the surface of the head 42 of the stopper 10,
- a safety factor $\gamma$ for the distance between the outer circle of protrusions 52b and the outer perimeter of the outer surface 46 of the head 42 of the stopper 10 and between adjacent circles of protrusions 52b, 52d, 52e, 52f, and
- the angular distance $\theta$ occupied by each protrusion, 52b, 52d, 52e, 52f.

The maximum allowed direct rigid coverage $\alpha$ is the maximum allowed contact area when the ends of two stoppers 10 are in direct contact with one another. For example, if the ends of the stoppers 10 are perfectly aligned, i.e., all protrusions 52 on one stopper 10 overlap perfectly with the protrusions 52 on the other stopper 10 and $\alpha=1$ (100%). When $\alpha = 0.5$, the contact area between the protrusions is 50% of the total contact area of the protrusions 52 of one of the stoppers 10.

The safety factor $\gamma$ is the radial spacing between concentric circles to avoid interpenetration of protrusions 52 radially.

If the outer surfaces 46 of the heads 42 of two stoppers 10 are perfectly aligned, direct outer surface to outer surface contact is present. In order to effectively reduce sticking between the two stoppers 10, the protrusions 52b, 52d, 52e, 52f cover a maximum of 50% of the surface area of the outer surface 46 of the head 42 of the stopper 10, i.e., $\alpha = 0.5$. When the outer diameter R of the head 42 of the stopper 10 is set at 1 (arbitrary units), the maximum total number $n_{max}$ of protrusions 52b, 52d, 52e, 52f for a given ratio $\beta$ of the radius r of the protrusions 52b, 52d, 52e, 52f and the outer diameter R of the head 42 of the stopper 10 (r/R) can be determined using the following expression:

$$\frac{n_{max}\pi(\beta R)^2}{\pi R^2} \leq \alpha \Leftrightarrow n_{max} \leq \frac{\alpha}{\beta^2}$$

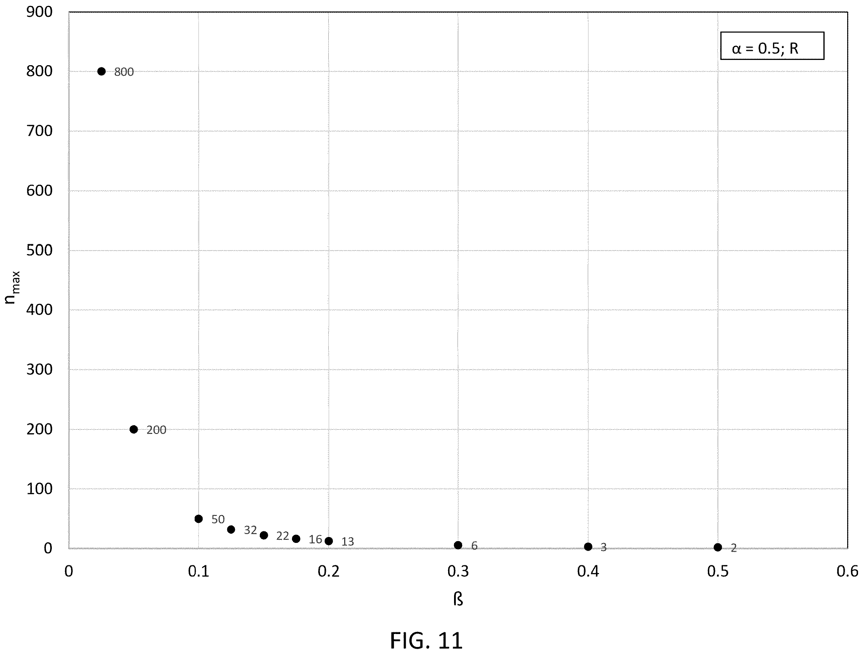

As an example, FIG. 11 shows the relationship between a ratio $\beta$ of 0.025-0.5 (2.5-50%) and the maximum total number $n_{max}$ of protrusions 52b, 52d, 52e, 52f when the maximum allowed direct rigid coverage $\alpha$ is 0.5 (50%) and the protrusions 52b, 52d, 52e, 52f have a radius r of 0.5.

In order to further minimize the area of contact, the protrusions 52b, 52d, 52e, 52f can be spaced apart in the radial direction, starting from a distance determined by the safety factor $\gamma$ from the outer perimeter of the stopper 10. Radially, the center of a given protrusion 52b will be located at a distance R - $\gamma$R - r from the center of the stopper 10. The safety factor $\gamma$ may be set at 0.05 (5 %). The angular distance $\theta_P$ occupied by one protrusion is:

$$\theta_P = tan^{-1}\frac{2\beta R}{R - \gamma R - \beta R}$$

To minimize contact between the outer surfaces 46 of the heads 42 of two stoppers 10 when the heads 42 of two stoppers 10 are perfectly aligned, the total angular distance occupied by the spaces between the protrusions is less than 180°. The maximum number $n_1$ of protrusions 52b in the outer circle adjacent the perimeter of the outer surface 46 of the head 42 of the stopper 10 as a function of the ratio $\beta$ may be determined as follows.

$$2\pi - n_a\theta < \pi \Leftrightarrow n_1 = int\left(\frac{\pi}{tan^{-1}\frac{2\beta R}{R - \gamma R - \beta R}}\right) + 1$$

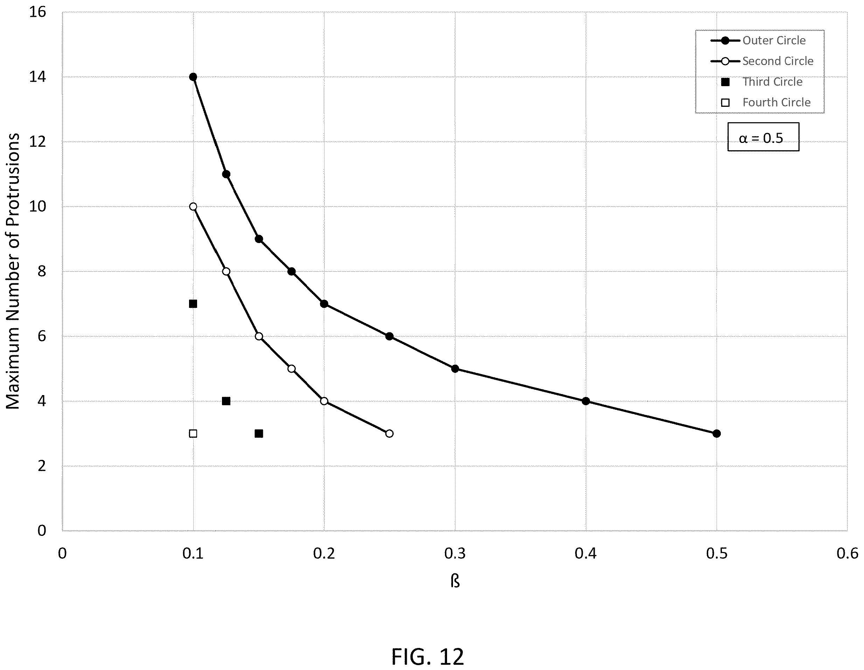

As an example, FIG. 12 shows the relationship between the ratio β of 0.1-0.5 (10-50%) and the maximum number $n_1$ of protrusions 52b in the outer circle adjacent the perimeter of the outer surface 46 of the head 42 of the stopper 10 when the maximum allowed direct rigid coverage α is 0.5 (50%) and the safety factor γ is 0.05 (5%).

As a specific example, for a maximum allowed direct rigid coverage α of 0.5 (50%) and a ratio β of 0.125 (12.5%), the maximum total number $n_{max}$ of protrusions is:

$$n_{max} = \frac{\alpha}{\beta^2} = \frac{0.5}{0.125^2} = 32$$

and the number $n_1$ of protrusions 52b placed on the outer circle adjacent the perimeter of the outer surface 46 of the head 42 of the stopper 10, when the head 42 of the stopper 10 has a radius R of 1 (arbitrary units) is:

$$n_1 = int\left(\frac{\pi}{tan^{-1}\frac{2\beta R}{R - \gamma R - \beta R}}\right) + 1 = int\left(\frac{\pi}{tan^{-1}\frac{2(0.125 \times 1)}{1 - (0.05 \times 1) - (0.125 \times 1)}}\right) + 1$$

$$= 11$$

The remaining 21 protrusions can be positioned in one or more concentric circles positioned within the outer circle adjacent the perimeter of the outer surface 46 of the head 42 of the stopper 10.

The distance between the concentric circles of protrusions 52 should be less than two times the diameter Dp of the protrusions 52, such that the space between the outer surfaces of two protrusions 52 in adjacent circles is less than the diameter Dp of the protrusions 52.

The equation for determining the maximum number $n_1$ of protrusions 52b on the circle adjacent the perimeter of the outer surface 46 of the head 42 of the stopper 10:

$$n_1 = int\left(\frac{\pi}{tan^{-1}\frac{2\beta R}{R - \gamma R - \beta R}}\right) + 1$$

can be used iteratively to determine the maximum number of protrusions 52d, 52e to place on each concentric inner circle by replacing R-γR -βR with $R_n$-γR-βR, where $R_n$ is the radius of the larger outer circle of protrusions adjacent the circle of protrusions for which the maximum number of protrusions is being determined.

As an example, FIG. 12 shows the relationship between a ratio β of 0.1-0.5 (10-50%) and the maximum number of protrusions on each circle of protrusions when the maximum allowed direct rigid coverage α is 0.5 (50%), the safety factor γ is 0.05 (5%), and the stopper has a radius R of 1 (arbitrary units).

As a specific example, when the maximum allowed direct rigid coverage α is 0.5 (50%), the safety factor γ is 0.05 (5%), the ratio β (r/R) is 0.1 (10%), the stopper has a radius R of 1 (arbitrary units), and the protrusions have a radius r of 0.1 (arbitrary units), the number $n_1$ of protrusions 52b placed on the outer circle of protrusions 52b adjacent the perimeter of the outer surface 46 of the head 42 of the stopper 10 is:

$$n_1 = int\left(\frac{\pi}{tan^{-1}\frac{2\beta R}{R - \gamma R - \beta R}}\right) + 1 = int\left(\frac{\pi}{tan^{-1}\frac{2(0.1 \times 1)}{1 - (0.05 \times 1) - (0.1 \times 1)}}\right) + 1 = 14$$

and $R_1$ is 0.75.

The number $n_2$ of protrusions 52d placed on the second concentric circle of protrusions 52d adjacent the outer circle of protrusions 52b is:

$$n_2 = int\left(\frac{\pi}{tan^{-1}\dfrac{2\beta R}{R_1 - \gamma R - \beta R}}\right) + 1 = int\left(\frac{\pi}{tan^{-1}\dfrac{2(0.1 \times 1)}{0.75 - (0.05 \times 1) - (0.1 \times 1)}}\right) + 1$$
$$= 10$$

and $R_2$ is 0.5.

The number $n_3$ of protrusions 52e placed on the third concentric circle of protrusions 52e adjacent the second concentric circle of protrusions 52d is:

$$n_3 = int\left(\frac{\pi}{tan^{-1}\dfrac{2\beta R}{R_2 - \gamma R - \beta R}}\right) + 1 = int\left(\frac{\pi}{tan^{-1}\dfrac{2(0.1 \times 1)}{0.5 - (0.05 \times 1) - (0.1 \times 1)}}\right) + 1$$
$$= 7$$

and $R_3$ is 0.25.

The number $n_4$ of protrusions 52f placed on the fourth concentric circle of protrusions 52f adjacent the third concentric circle of protrusions 52e is:

$$n_4 = int\left(\frac{\pi}{tan^{-1}\dfrac{2\beta R}{R_3 - \gamma R - \beta R}}\right) + 1 = int\left(\frac{\pi}{tan^{-1}\dfrac{2(0.1 \times 1)}{0.25 - (0.05 \times 1) - (0.1 \times 1)}}\right) + 1$$
$$= 3$$

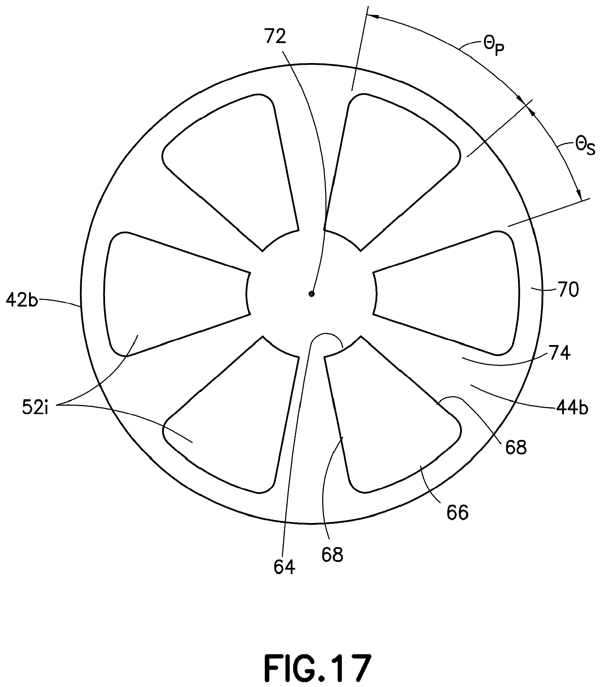

The effective total surface area of the protrusions 52b, 52d, 52e, 52f on the head 42 of the stopper 10 can be calculated by summing the total number ($n_1 + n_2 + n_3$, etc.) of protrusions 52b, 52d, 52e, 52f for a given ratio $\beta$ as shown in FIG. 17, for a given maximum allowed direct rigid coverage $\alpha$ and safety factor $\gamma$.

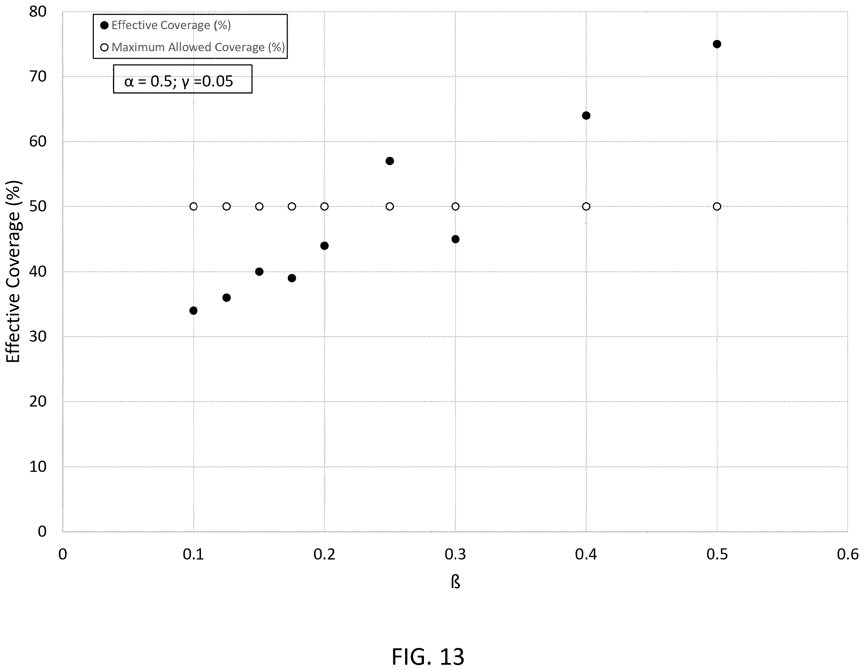

As an example, FIG. 13 shows the effective total contact area for ratios $\beta$ of 0.1-0.5 (10-50%) when the maximum allowed direct rigid coverage $\alpha$ is 0.5 (50%), and the safety factor $\gamma$ is 0.05 (5%).

As a specific example, when the ratio $\beta$ of 0.1 (10%), the maximum allowed direct rigid coverage $\alpha$ is 0.5 (50%), the safety factor $\gamma$ is 0.05 (5%), the radius R of the outer surface 46 of the head 42 of the stopper 10 is 1 (arbitrary units), and the radius r of the protrusions is 0.1 (arbitrary units), the total number N of protrusions is 34 as calculated above, the effective total contact area is:

$$\frac{\pi N r^2}{\pi R^2} \times 100 = \frac{\pi \times 34 \times 0.1^2}{\pi \times 1^2} \times 100 = 34\%$$

which is below the maximum allowed direct rigid coverage $\alpha$ of 50%. As can be seen from FIG. 13, for ratios $\beta$ (r/R) of 0.3 or less, the effective total contact area is less than or close to the maximum allowed direct rigid coverage $\alpha$ of 50%.

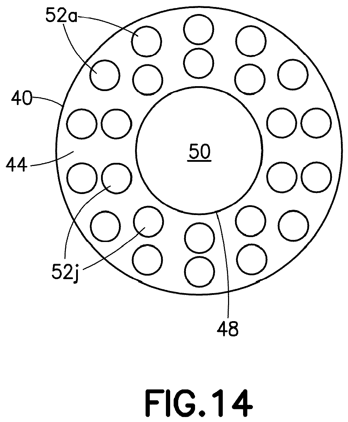

The equations discussed above for determining the placement of the protrusions 52b, 52d, 52e, 52f on the outer surface 46 of the head 42 of a stopper 10 can also be applied to the protrusions 52a provided on the outer surface 44 of the tail 40 of a stopper 10 with the equations for the outer circle adjacent the perimeter of the outer surface 46 of the head 42 of the stopper 10 being used when the radial width of the annular ring defining the outer surface 44 of the tail 40 of the stopper 10 and the diameter of the protrusions 52a only allows for one ring of protrusions 52a to be provided. When the radial width of the annular ring defining the outer surface 44 of the tail 40 of the stopper 10 and the diameter of the protrusions allows for more than one circle of protrusions 52a, 52j to be provided (FIG. 14), the additional equations may be used for the protrusions on the outer surface 44 of the tail 40 of the stopper 10.

The inventive stopper may have several embodiments including but not limited to:

(1) a single outer circle of protrusions provided on the outer surface of the head of the stopper adjacent the perimeter of the outer surface of the head of the stopper with an additional protrusion provided in the center of the outer surface of the head of the stopper (FIG. 6);

(2) more than one circle of protrusions provided on the outer surface of the head of the stopper, the circles being concentric and each circle having a different number of protrusions, where the closer the circle is to the center of the outer surface of the head of the stopper, the lower the number of protrusions provided in the circle (FIG. 10);

(3) more than one circle of protrusions provided on the outer surface of the head of the stopper, the circles being concentric and each circle having a different number of protrusions, where the closer the circle is to the center of the outer surface of the head of the stopper, the lower the number of protrusions provided in the circle and an additional protrusion provided in the center of the outer surface of the head of the stopper; (4) a single outer circle of protrusions provided on the outer surface of the tail of the stopper between the outer perimeter of the outer surface of the tail of the stopper and the outer perimeter of the opening of the cavity (FIG. 3); (5) more than one circle of protrusions provided on the outer surface of the end of the stopper between the outer perimeter of the outer surface of the tail of the stopper and the outer perimeter of the opening of the cavity, the rings being concentric and each ring having a different number of protrusions, where the closer the ring is to the outer perimeter of the cavity, the lower the number of protrusions provided in the ring (FIG. 14); and (6) any combination of the head configurations (1-3) with any of the tail configurations (4 and 5). Further, for any combination of the head configurations (1-3) with any of the tail configurations (4 and 5), the head may have a different total number of protrusions than the tail, and/or the number of protrusions in the outer ring of protrusions on the head may be different from the number of protrusions in the outer ring of protrusions on the tail, and/or the size of the protrusions on the head may be different from the size of the protrusions in the outer ring of protrusions on the tail. For example, the outer ring of protrusions on the head may include 7 protrusions while the outer ring of protrusions on the tail may include 6 protrusions.

The outer surface of the head 42 of the stopper 10 may be flat, convex, or conical. A convex outer surface of the head 42 of the stopper 10 is one in which the outer surface of the head of the stopper is curved, such that the apex of the curvature extends away from a plane which is orthogonal to the longitudinal axis of the stopper and comprising the outer perimeter of the outer surface, i.e., a distance between the apex of the outer surface of the head and the plane is greater than the distance between the portion of the outer surface of the head adjacent the outer perimeter of the outer surface of the head and the plane when the stopper is viewed from the side in a direction perpendicular to the longitudinal axis of the stopper.

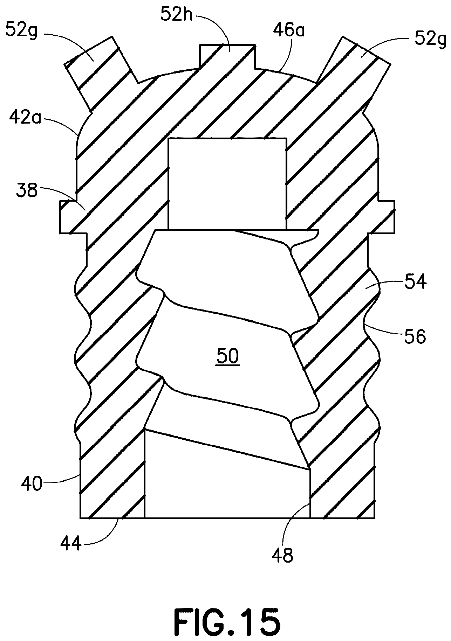

When the outer surface 46a of the head 42a of the stopper 10 is convex (FIG. 15), any additional protrusions 52g placed inside of the outer ring of protrusions 52h adjacent the perimeter of the outer surface 46a of the head 42a of the stopper 10 may have a height in a direction extending outwardly and orthogonally from the outer surface 46a of the head 42a of the stopper 10 that is less than a height in a direction extending outwardly from the outer surface 46a of the head 42a of the stopper 10 of the protrusions 52h in the outer ring of protrusions 52g adjacent the perimeter of the outer surface 46a of the head 42 of the stopper 10.

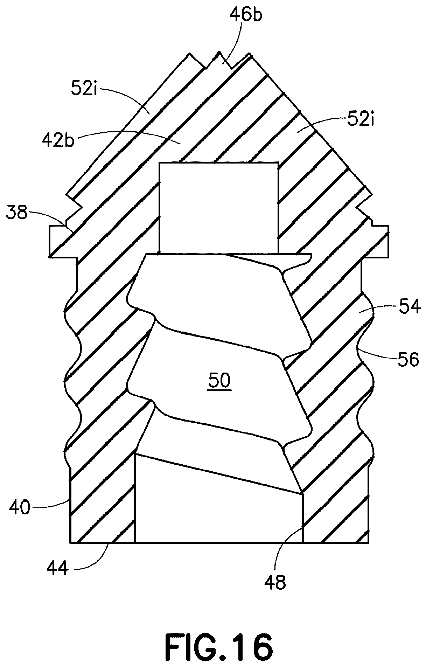

When the outer surface 46b of the head 42b of the stopper 10 is conical (FIG. 16), as an alternative to the configurations of protrusions discussed above, a single ring of protrusions 52i may be provided.

The protrusions 52i may have a substantially triangular shape, preferably a shape that is substantially an isosceles triangle, with a vertex 64 corresponding to the vertex angle of the triangular shape of the protrusion 52i, a base 66 corresponding to the base of the triangular shape of the protrusion 52i, and two sides 68a, 68b extending between the vertex 64 and the base 66. The vertex 64 of the protrusion 52i has a circumferential width that is less than the circumferential width of the base 66 of the protrusion 52i.

The vertex 64 of each protrusion 52i is adjacent the center of the outer surface 46 of the head 42 of the stopper 100, i.e., the center of origin of the outer perimeter of the outer surface 46 of the head 42 of the stopper 100, which corresponds to the apex of the cone of the head 42 of the stopper 100, and the base 66 of each protrusion 52i is adjacent the outer perimeter of the outer surface 46 of the head 42 of the stopper 100.

The base 66 of the protrusions 52i may be rounded to correspond to the round outer perimeter of the outer surface 46 of the head 42 of the stopper 100. A space 70 may be provided between the base 66 of the protrusions 52i and the outer perimeter of the outer surface 46 of the head 42 of the stopper 100, such that the protrusions 52i are distanced from the outer perimeter of the outer surface 46 of the head 42 of the stopper 100, and an annular portion of the outer surface 46 of the head 42 of the stopper 100 directly adjacent to the outer perimeter of the outer surface 46 of the head 42 of the stopper 100 is not covered by the protrusions 52i.

The vertex 64 of the protrusions 52i may be truncated, such that a space 72 is provided at the center of the outer surface 46 of the head 42 of the stopper 100. The space 72 may be circular and the truncated vertex 64 of the protrusions 52i may be curved such that the vertices 64 of the protrusions 52i define the space 72 at the center of the outer surface 46 of the head 42 of the stopper 100 that is not covered by the protrusions 52i.

The protrusions 52i may be evenly distributed around the circumference of the outer surface 46 of the head 42 of the stopper 100 such that corresponding substantially triangular spaces 74 are provided between adjacent protrusions 52i. Again, as with all of the embodiments discussed above, the angular distance $\theta_S$ between two adjacent protrusions is less than the angular distance $\theta_P$ occupied by one of the protrusions, i.e., $\theta_S \leq \theta_P$.

Sticking of the outer sidewalls of the stopper body can also be a problem. Therefore, anti-sticking features may also be included between annular ribs provided on the outer sidewall of the stopper body.

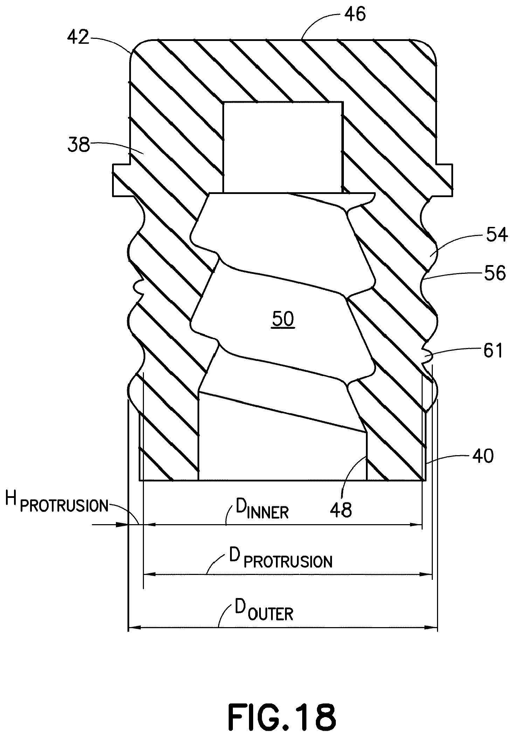

As shown in FIG. 18, the substantially cylindrical stopper body 38 may include a plurality of annular ribs 54 around the circumference of the outer sidewall 58 of the stopper body 38. The ribs 54 may be evenly spaced from one another by grooves 56. The ribs 54 have a diameter $D_{outer}$ that is greater than the diameter Dinner of the grooves 56.

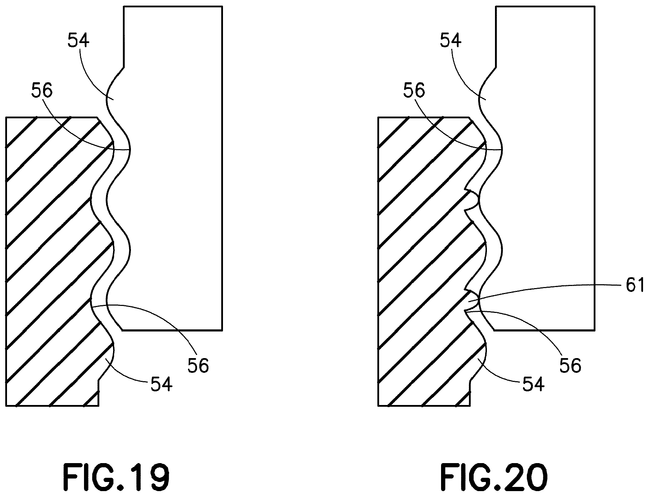

When the outer sidewalls 58 of two stoppers 100 come in contact with one another, the ribs 54 of one stopper may be received in the grooves 56 of the other stopper 100 and vice versa resulting in full contact between the outer sidewalls 58 of the stoppers 100 (FIG. 19).

In order to avoid such contact, a plurality of protrusions 61 extend outwardly from the outer sidewall 58 of the stopper body 38 in a radial direction from within at least one of the grooves 56 (FIG. 20). By placing the protrusions 61 within the grooves 56, it is possible to reduce sticking without interfering with the functionality of the ribs 54, which provide a seal with inner sidewall of the syringe body 14 while the grooves 56 reduce the force needed to move the stopper 100 within the chamber 24 of the syringe 12.

The height $H_{protrusion}$ of the protrusions 61 extending radially outward from the groove 56 is set such that the height $H_{protrusion}$ is less than the diameter $D_{outer}$ of the stopper body 38 in the area of the ribs 54 minus the diameter Dinner of the stopper body in the area of the groove 56 divided by 2, i.e., $H_{protrusion} < (D_{outer} - D_{inner})/2$. The diameter $D_{protrusion}$ of the stopper body 38 in the area of the protrusion 61 is the sum of the diameter Dinner of the stopper body in the area of the groove 56 and the height $H_{protrusion}$ of the. A safety factor may be included to assure that $H_{protrusion} < (D_{outer} - D_{inner})/2$.

Monte Carlo simulations using Oracle™ Crystal Ball software (version 11.1.2.4.600) in Microsoft™ Excel were conducted to determine the appropriate number (spacing) of the protrusions 61 around the circumference of the stopper 100 and height of the protrusions 61 to provide the desired anti-sticking properties. The simulations used the following factors:

- the diameter $D_{outer}$ of the stopper body 38 in the area of the ribs 54;
- the diameter Dinner of the stopper body 38 in the areas of the grooves 56;
- the diameter $D_{protrusion}$ of the stopper body 38 in the area of the protrusion 61;
- the percentage of deformation $f_{deformation}$ due to ovalization when the stoppers 100 are placed under a force that can cause sticking;
- the height $H_{protrusion}$ of the protrusions 61; and
- a safety factor $f_p$ for the height of the protrusion.

The groove 56 having diameter Dinner was represented by an ellipse having a great axis $a_{inner}$ and small axis $b_{inner}$ centered on the origin (0, 0) of a cartesian coordinate system (x,y) by:

$$\left(\frac{D_{inner}}{2}\right)^2 = \left(\frac{x}{a_{inner}}\right)^2 + \left(\frac{y}{b_{inner}}\right)^2 \quad \text{(Equation 1)}$$

Similarly, the rib 54 having diameter $D_{outer}$ was represented by an ellipse centered at $\left(\frac{D_{inner}+D_{outer}}{2}, 0\right)$ and having a great axis $a_{outer}$ and small axis $b_{outer}$ by:

$$\left(\frac{D_{outer}}{2}\right)^2 = \left(\frac{x}{a_{outer}} - \left(\frac{D_{inner} + D_{outer}}{2}\right)\right) \left(\frac{x}{a_{outer}} - \left(\frac{D_{inner} + D_{outer}}{2}\right)\right)$$

$$+ \left(\frac{y}{b_{outer}}\right)^2 \quad \text{(Equation 2)}$$

When no external force is applied ($f_{deformation} = 0$), for each case, both ellipses are circles and the small axis is equal to the great axis.

By switching to polar coordinates with $x = r\cos\theta$ and $x = r\sin\theta$, Equation 1 becomes:

$$r = \frac{\frac{D_{inner}}{2}}{\sqrt{\frac{cos^2\theta}{\frac{a_{inner}^2}{2}} + \frac{sin^2\theta}{\frac{b_{inner}^2}{2}}}} \quad \text{(Equation 3)}$$

By dividing the right member of Equation 3 by $1/a_{inner}$, the equation for the groove ellipse can be obtained where $a_{inner} = \frac{D_{inner}}{2}\left(1 - \frac{f_{deformation}}{100}\right)$ and $b_{inner} = \frac{1}{a_{inner}}$ (isotropic deformation under constraint):

$$r_{inner}(\theta) = \frac{\frac{D_{inner}}{2} a_{deformation}}{\sqrt{cos^2\theta + \frac{a_{inner}}{b_{inner}} sin^2\theta}} \quad \text{(Equation 4)}$$

Similarly, switching Equation 2 for the rib ellipse to polar coordinates, with similar relationships as for the groove ellipse, $a_{outer} = \frac{D_{outer}}{2}\left(1 - \frac{f_{deformation}}{100}\right)$ and $b_{outer} = \frac{1}{a_{outer}}$ :

$$r_{outer}^2 \left(cos^2\theta + \frac{a_{outer}^2}{b_{outer}^2}\right) - 2r_{outer}a_{outer}\left(\frac{D_{inner} + D_{outer}}{2}\right)cos\theta + \frac{a_{outer}^2 D_{outer}}{2}$$

$$* (D_{outer}/2 - 2D_{inner}/2) \quad \text{(Equation 5)}$$

Positive roots $r_{outer,1}(\theta)$ and $r_{outer,2}(\theta)$ of this equation represent the envelope of the rib ellipse, and for a given $\theta$, $d(\theta)$, the distance between the two ellipses is represented by:

$$d(\theta) = min\left(r_{outer,1}(\theta), r_{outer,2}(\theta)\right) - r_{inner}(\theta) \quad \text{(Equation 6)}$$

The minimum angular distance between two protrusions 61 was approximated for a given $\theta$ by the condition, $\Delta\theta$ being a 1° increment used during simulation:

$$d(\theta) < H_{protrusion}$$

$$d(\theta + \Delta\theta) > H_{protrusion}$$

Also,

$$H_{protrusion} = \left( \frac{D_{outer} - D_{inner}}{2} \right) / f_p$$

For the simulations, the following rib diameters $D_{outer}$ and groove diameters $D_{inner}$ were used.

| Stopper | $D_{outer}$ nominal (mm) | $D_{outer}$ tolerance (mm) | $D_{inner}$ nominal (mm) | $D_{inner}$ tolerance (mm) |
|---|---|---|---|---|
| A | 5.25 | 0.2 | 4.2 | 0.2 |
| B | 6.9 | 0.15 | 6 | 0.2 |
| C | 6.6 | 0.1 | 6.05 | 0.1 |
| D | 9.15 | 0.15 | 8 | 0.2 |
| E | 8.9 | 0.1 | 8.3 | 0.15 |
| F | 11.2 | 0.3 | 10 | 0.2 |
| G | 15 | 0.1 | 13.7 | 0.25 |
| H | 20.05 | 0.2 | 18.5 | 0.2 |

The isotropic ovalization parameter $f_{deformation}$ was varied from 0% (no ovalization) to 10% (extreme ovalization, unlikely to happen in real world conditions). The protrusion height $H_{protrusion}$ was varied from $0.5 * (D_{outer}/2 - D_{inner}/2)$ to $(D_{outer}/2 - D_{inner}/2)$, i.e., from 50% of the depth of the groove 56 to 100% of the depth of the groove 56.

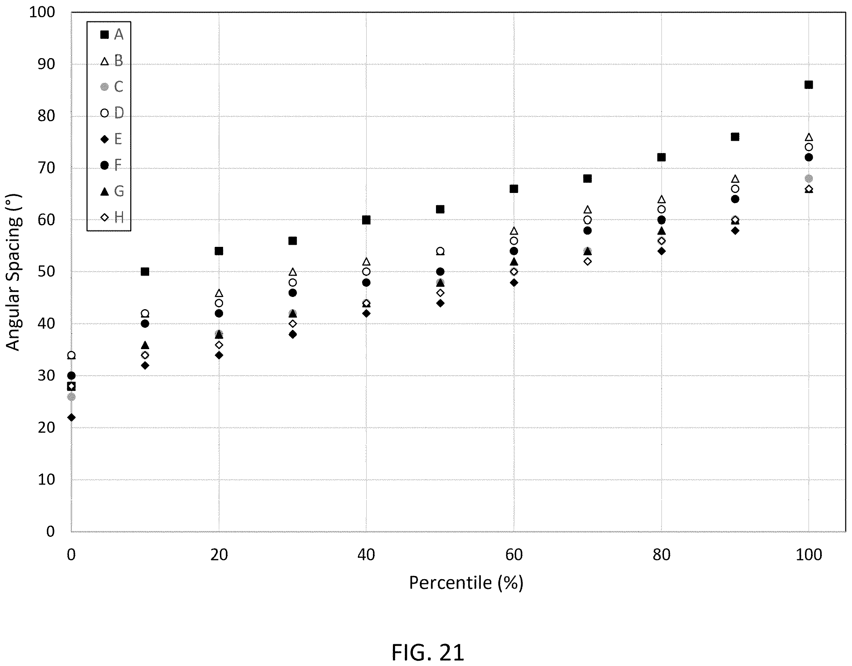

The Monte Carlo simulation was run conservatively with 10,000 trials per configuration and the determined minimum angular distance between protrusions 61 for each stopper are shown in FIG. 21.

A finite element analysis (FEA) was undertaken in order to determine the possible effects of the protrusions 61 on the stopper properties, because protrusions 61 inside ribs could impact stopper functionalities such as activation and gliding forces, or container closure integrity if contact pressure between the barrel and the stopper is modified.

A 1mL barrel (inner diameter of 6.35 mm) was assembled with a 1 mL stopper with protrusions having a height equal to about 50% of the distance between the inner groove and the outer diameter of the rib. Six protrusions 61 were disposed 60° apart in each groove with a 30° offset between each consecutive groove. FEA analysis of the system has shown that inner surface of the barrel to external surface of the protrusions was positive, and thus that no direct contact between the protrusions and the inner surface of the barrel was observed. Contact pressures of the stopper were indeed not modified, and thus functionality of the stopper was not impacted by the protrusions.

As can be seen in FIG. 21, the angular spacing of the protrusions 61 is similar regardless of the stopper design with a minimum of about 30°, a median of 40°-60°, and a maximum of 60°-80°. Based on the median values, 6-9 protrusions 61 are provided on the circumference of the stoppers to avoid sticking.

Protrusions 61 may be provided in more than one groove with an offset spacing from groove 56 to groove 56 corresponding to half the angular distance between the protrusions in order to further minimize the area of contact. For example, with 6 protrusions per groove 56, the first groove 56 will have protrusions at 0, 60°, 120°, 180°, 240°, and 300° around the circumference of the stopper 100, the second groove 56 will have protrusions 61 at 30°, 90°, 150°, 210°, 270°, and 330° around the circumference of the stopper 100, and so on.

The height of the protrusions 61 may be from 50% to less than 100% of the difference between the rib radius $D_{outer}/2$ and the groove radius Dinner/2.

The protrusions 61 may be rounded having a convex outer surface and may have a substantially oval cross-section or may have any shape as long as the shape and dimensions of the protrusions 61 provides for anti-sticking of the outer sidewall 58 of one stopper 100 to the outer sidewall 58 of another stopper 100. For example, the protrusions 61 may have a cross-section that is circular, oval, square, rectangular, or polygonal, and may have a flat outer surface or a rounded outer surface with straight sides. The rounded convex surface of the protrusions may be convex in all directions such that the protrusion has a dome shape.

The stopper can be made of any suitable material for providing for easy movement of the stopper within the syringe barrel while still providing a seal between the stopper and the syringe barrel. Such materials include, but are not limited to, natural rubber, synthetic rubber, and more particularly rubbers made from butyl, bromobutyl, chlorobutyl, silicone, nitrile, styrene butadiene, polychlororprene, ethylene propylene diene, fluoroelastomers, thermoplastic elastomers, and combinations and blends thereof, and is preferably made from butyl rubber, such as bromobutyl. The outer surface of the stopper may optionally be coated with a suitable coating material. Such coating materials include but are not limited to coatings that act as a barrier between the stopper material and the pharmaceutical composition that occupies the chamber 24 of the syringe 12 and enables reduction of extraction and/or leaching of substances from the stopper material and infiltration of the pharmaceutical composition into the stopper material, for example, a fluoropolymer such as polytetrafluoroethylene (PTFE), ethylene tetrafluoroethylene (ETFE), expanded polytetrafluoroethylene (ePTFE), flurorinated ethylene propylene (FEP), polyvinylidene fluoride, polyvinylfluoride, perfluoropropylvinylether, perfluoroalkoxy polymers, tetrafluoroethylene (TFE), parylene, or non-fluoropolymers such as polyethylene, polypropylene, Parylene C, and Parylene F, and/or a silicone layer for lubricity, decreased stickiness, and/or protection of the stopper during vent tube assembly processing, for example,

Whereas particular aspects of this invention have been described above for purposes of illustration, it will be evident to those skilled in the art that numerous variations of the details of the present invention may be made without departing from the invention.

**Claims**

1. A stopper for a syringe comprising:

    a body having a proximal tail adapted for attachment to a plunger rod of the syringe and a distal head; and a plurality of protrusions extending from an outer surface of the tail and/or the head,
    **characterized in that**:
    the protrusions are arranged in at least one circle on the outer surface of the tail and/or the head of the stopper and an angular distance between two adjacent protrusions in the circle is less than the angular distance occupied by one of the protrusions.

2. The stopper of claim 1, wherein a cavity adapted to receive and engage at least a portion of the plunger rod of the syringe is defined within the body of the stopper and an opening to the cavity is provided in the tail of the stopper, and protrusions are provided on the tail of the stopper and are arranged in at least one circle on the outer surface of the tail of the stopper extending from an annular ring on the outer surface of the tail of the stopper defined between an outer perimeter of the end face and an outer perimeter of the cavity.

3. The stopper of claim 1 or claim 2, wherein the protrusions are arranged in an outer circle around an outer perimeter of the outer surface of the tail and/or the head of the stopper and further in at least one additional concentric circle within the outer circle, and an angular distance between two adjacent protrusions in each circle is less than the angular distance occupied by one of the protrusions.

4. The stopper of any of claims 1-3, wherein a first circle of substantially cylindrical protrusions is provided on the tail and/or the head of the stopper adjacent an outer perimeter of the outer surface of the tail and/or the head of the stopper and a number $n_1$ of protrusions provided in the first circle is defined by:

$$n_1 > int\left(\frac{\pi}{tan^{-1}\dfrac{2\beta R}{R - \gamma R - \beta R}}\right) + 1$$

    where R is an outer diameter of the outer surface of the tail and/or the head of the stopper, $\beta$ is a ratio of a radius r of each protrusion and the radius R (r/R), and $\gamma$ is a safety factor for a distance between the circle of protrusions and an outer perimeter of the end surface of the head of the stopper.

5. The stopper of claim 4, wherein at least one concentric circle of protrusions is provided within the first circle of protrusions and a number $n_i$ of protrusions provided in the $i^{th}$ circle is defined by:

$$n_i > int\left(\frac{\pi}{tan^{-1}\dfrac{2\beta R}{R_i - \gamma R - \beta R}}\right) + 1$$

    where $R_i$ is the inner diameter of the i-1 circle of protrusions.

6. The stopper of claim 2, wherein a single circle of substantially cylindrical protrusions is provided and a minimum number of protrusions provided in the circle is defined by:

$$n_{min} = int\left(\frac{\pi}{\theta_P}\right) + 1$$

where

$$\theta_P = 2tan^{-1}\frac{D_P}{\frac{1}{2}(D_O + D_C)}$$

where Dp is a diameter of each protrusion, $D_O$ is an outer diameter of the outer surface of the tail of the stopper and $D_C$ is a diameter of the outer perimeter of the cavity; and
wherein the angular distance between protrusions is defined by:

$$\theta_S = (2\pi - (\theta_P \times n_{min}))/n_{min}$$

7. The stopper of claim 1, 2, or 6, wherein protrusions are arranged in an outer circle around an outer perimeter of the outer surface of the head of the stopper and with a protrusion within the outer circle positioned in a center of the outer surface of the head of the stopper.

8. The stopper of any of claims 1-7, wherein the protrusions are equally spaced around each circle, the protrusions are substantially cylindrical, and/or the outer surface of the head of the stopper is flat, convex, or conical.

9. The stopper of any of claims 1-8, wherein the head of the stopper is convex and protrusions in a circle adjacent an outer perimeter of the outer surface of the head of the stopper have a height in a direction extending outwardly from the outer surface of the head of the stopper that is greater than a height in a direction extending outwardly from the outer surface of the head of the stopper of protrusions located inside the circle adjacent an outer perimeter of the outer surface of the head of the stopper.

10. The stopper of claim 1 or claim 2, wherein the head of the stopper is conical and a circle of protrusions having a substantially triangular shape, preferably a shape that is substantially an isosceles triangle, with a vertex corresponding to the vertex angle of the triangular shape of the protrusion, a base corresponding to the base of the triangular shape of the protrusion , and two sides extending between the vertex and the base extend from an outer surface of the head of the stopper.

11. A stopper for a syringe comprising:

a body having a proximal tail adapted for attachment to a plunger rod of the syringe, a distal head, a plurality of annular ribs around the circumference of an outer sidewall of the body, and a plurality of grooves around the circumference of an outer sidewall of the body, wherein the ribs are spaced from one another by the grooves;
**characterized in that**:
a plurality of protrusions extend outwardly from the outer sidewall of the body in a radial direction from within at least one of the grooves.

12. The stopper of claim 11, wherein an angular spacing of the protrusions within the at least one groove is 30° or more, a median angular spacing of the protrusions within the at least one groove is 40°-60°, and/or a maximum angular spacing of the protrusions within the at least one groove is 60°-80°.

13. The stopper of claim 11 or claim 12, wherein protrusions are provided in more than one groove with an offset spacing from groove to groove corresponding to half the angular distance between the protrusions in each groove.

14. The stopper of any of claims 11-13, wherein a height of the protrusions in the radial direction is 50% to less than 100% of the difference between a radius of the rib and a radius of the groove.

15. A syringe comprising:

    a syringe body defining a chamber;
    a plunger rod at least partially received within the chamber; and
    a stopper according to any of claims 1-14 attached to an end of the plunger rod received within the chamber.

FIG.1

FIG.2

**FIG.3**

**FIG.4**

FIG.5

**FIG.6**

**FIG.7**

FIG.8

FIG.9

FIG.10

EP 4 190 378 A1

FIG. 11

FIG. 12

FIG. 13

FIG.14

FIG.15

46b

52i

42b

38

50

54

56

40

44

48

FIG.16

FIG.17

FIG.18

FIG.19

FIG.20

FIG. 21

Europäisches
Patentamt

European
Patent Office

Office européen
des brevets

# EUROPEAN SEARCH REPORT

**Application Number**

EP 21 21 2446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 684 576 A2 (BECTON DICKINSON CO [US]) 15 January 2014 (2014-01-15) * paragraph [0041] - paragraph [0046]; figures 8, 9 * | 1,3-7,9 | INV. A61M5/315 A61J1/06 |
| X | EP 2 660 163 A1 (NIPRO CORP [JP]) 6 November 2013 (2013-11-06) * paragraph [0019] - paragraph [0025]; figures 2b, 3, 4 * | 1,8-10, 15 | |
| X | US 2015/025454 A1 (WETZEL LANCE W [US] ET AL) 22 January 2015 (2015-01-22) * paragraph [0074] - paragraph [0079]; figure 3A * | 11,15 | |
| X | US 2020/139043 A1 (SWANTNER MICHAEL [US] ET AL) 7 May 2020 (2020-05-07) * paragraph [0160] - paragraph [0186]; figures 7A/8A, 15C/D * | 1,11-15 | |
| X | WO 2005/099793 A1 (LILLY CO ELI [US]; DAI HE [US]; ALLEN DOUGLAS JAMES [US]) 27 October 2005 (2005-10-27) * page 3 - page 6; figures 3, 6 * | 1,2 | TECHNICAL FIELDS SEARCHED (IPC) A61M A61J |

The present search report has been drawn up for all claims

| Place of search | Date of completion of the search | Examiner |
|---|---|---|
| Munich | 27 May 2022 | Filippi, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 21 21 2446**

27-05-2022

| Patent document cited in search report | | | Publication date | Patent family member(s) | | | Publication date |
|---|---|---|---|---|---|---|---|
| EP 2684576 | A2 | | 15-01-2014 | BR | PI0614088 | A2 | 09-03-2011 |
| | | | | BR | 122017025057 | B1 | 07-04-2020 |
| | | | | CN | 101282753 | A | 08-10-2008 |
| | | | | EP | 1917056 | A1 | 07-05-2008 |
| | | | | EP | 2684576 | A2 | 15-01-2014 |
| | | | | EP | 3156090 | A1 | 19-04-2017 |
| | | | | ES | 2609474 | T3 | 20-04-2017 |
| | | | | ES | 2731323 | T3 | 15-11-2019 |
| | | | | MX | 343972 | B | 30-11-2016 |
| EP 2660163 | A1 | | 06-11-2013 | CN | 103269955 | A | 28-08-2013 |
| | | | | EP | 2660163 | A1 | 06-11-2013 |
| | | | | JP | 5768820 | B2 | 26-08-2015 |
| | | | | JP | WO2012090328 | A1 | 05-06-2014 |
| | | | | US | 2013270271 | A1 | 17-10-2013 |
| | | | | WO | 2012090328 | A1 | 05-07-2012 |
| US 2015025454 | A1 | | 22-01-2015 | AU | 2014290079 | A1 | 11-02-2016 |
| | | | | AU | 2014290081 | A1 | 11-02-2016 |
| | | | | AU | 2014290084 | A1 | 11-02-2016 |
| | | | | CA | 2918482 | A1 | 22-01-2015 |
| | | | | CA | 2919150 | A1 | 22-01-2015 |
| | | | | CA | 2919154 | A1 | 22-01-2015 |
| | | | | CN | 105530976 | A | 27-04-2016 |
| | | | | CN | 105530977 | A | 27-04-2016 |
| | | | | CN | 105592875 | A | 18-05-2016 |
| | | | | CN | 110917450 | A | 27-03-2020 |
| | | | | DK | 3021915 | T3 | 14-06-2018 |
| | | | | DK | 3021916 | T3 | 06-06-2018 |
| | | | | DK | 3021917 | T3 | 22-05-2018 |
| | | | | EP | 3021915 | A2 | 25-05-2016 |
| | | | | EP | 3021916 | A1 | 25-05-2016 |
| | | | | EP | 3021917 | A1 | 25-05-2016 |
| | | | | EP | 3431124 | A2 | 23-01-2019 |
| | | | | JP | 6574767 | B2 | 11-09-2019 |
| | | | | JP | 2016524990 | A | 22-08-2016 |
| | | | | JP | 2019162534 | A | 26-09-2019 |
| | | | | KR | 20160039207 | A | 08-04-2016 |
| | | | | KR | 20160088852 | A | 26-07-2016 |
| | | | | KR | 20160088853 | A | 26-07-2016 |
| | | | | PT | 3021915 | T | 14-06-2018 |
| | | | | PT | 3021916 | T | 28-05-2018 |
| | | | | PT | 3021917 | T | 07-05-2018 |
| | | | | US | 2015025454 | A1 | 22-01-2015 |
| | | | | US | 2015025455 | A1 | 22-01-2015 |
| | | | | US | 2015025456 | A1 | 22-01-2015 |

EPO FORM P0459

## ANNEX TO THE EUROPEAN SEARCH REPORT
## ON EUROPEAN PATENT APPLICATION NO.

EP 21 21 2446

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2022

| Patent document cited in search report | Publication date | Patent family member(s) | | Publication date |
|---|---|---|---|---|
| | | US | 2017157328 A1 | 08-06-2017 |
| | | WO | 2015009866 A2 | 22-01-2015 |
| | | WO | 2015009868 A1 | 22-01-2015 |
| | | WO | 2015009871 A1 | 22-01-2015 |
| US 2020139043 A1 | 07-05-2020 | AU | 2016344179 A1 | 12-04-2018 |
| | | AU | 2021257891 A1 | 18-11-2021 |
| | | BR | 112018008550 A2 | 23-10-2018 |
| | | CA | 3003323 A1 | 04-05-2017 |
| | | CL | 2018001079 A1 | 31-08-2018 |
| | | CN | 108348693 A | 31-07-2018 |
| | | CN | 113082348 A | 09-07-2021 |
| | | DK | 3368109 T3 | 16-08-2021 |
| | | EP | 3368109 A1 | 05-09-2018 |
| | | EP | 3884975 A1 | 29-09-2021 |
| | | ES | 2883268 T3 | 07-12-2021 |
| | | HK | 1253566 A1 | 21-06-2019 |
| | | HU | E055239 T2 | 29-11-2021 |
| | | IL | 258989 A | 30-06-2021 |
| | | JP | 6943850 B2 | 06-10-2021 |
| | | JP | 2018531735 A | 01-11-2018 |
| | | JP | 2022000170 A | 04-01-2022 |
| | | KR | 20180075516 A | 04-07-2018 |
| | | PH | 12018500918 A1 | 05-11-2018 |
| | | PL | 3368109 T3 | 27-12-2021 |
| | | PT | 3368109 T | 19-08-2021 |
| | | RU | 2018119343 A | 28-11-2019 |
| | | TW | 201722492 A | 01-07-2017 |
| | | US | 9480797 B1 | 01-11-2016 |
| | | US | 2017119963 A1 | 04-05-2017 |
| | | US | 2020139043 A1 | 07-05-2020 |
| | | WO | 2017075303 A1 | 04-05-2017 |
| | | ZA | 201803474 B | 29-01-2020 |
| WO 2005099793 A1 | 27-10-2005 | EP | 1735032 A1 | 27-12-2006 |
| | | JP | 2007532181 A | 15-11-2007 |
| | | US | 2007219507 A1 | 20-09-2007 |
| | | WO | 2005099793 A1 | 27-10-2005 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PENOCCHIO et al.** Structural Features of the Carbon- Sulfur Chemical Bond: A Semi-Experimental Perspective. *Can. J. Chem.,* 2016, vol. 94 (12), 1065-1076
- **LSRAELACHVILI.** *Intermolecular and Surfaces Forces,* 2003
- **POPOV ; RIGOROUS TREATMENT OF CONTACT PROBLEMS - HERTZIAN CONTACT.** *Contact Mechanics and Friction,* 2010, 55-70

- Johnson Kendall Roberts theory, Surface Energy and the Contact of Elastic Solids. *Proc. R. Soc. London Math. Phys. Sci.,* 1971, vol. 324 (1558), 301-313
- Multi Contact Mechanics, Persson, Theory of Rubber Friction and Contact Mechanics. *J. Chem. Phys.,* 2001, vol. 115 (8), 3840-3861